# EUROPEAN PATENT SPECIFICATION

(11) **EP 1 395 254 B1**
(45) Date of publication and mention of the grant of the patent: **04.04.2007**
(21) Application number: 02727063.6
(22) Date of filing: 11.06.2002
(51) Int. Cl.: A61K 31/22, A61K 31/365, A61K 9/48, A61K 47/44

(54) **ORAL PHARMACEUTICAL COMPOSITION CONTAINING A STATIN DERIVATIVE**
ORAL ANZUWENDENDE ARZNEIZUSAMMENSETZUNG ENTHALTEND EIN STATINDERIVAT
COMPOSITION PHARMACEUTIQUE ORALE COMPRENANT UN DERIVE DE STATINE

(30) Priority: 12.06.2001 BE 200100098
(43) Date of publication of application: 10.03.2004
(73) Proprietor: Galephar M/F, 6900 Marche-en-Famenne (BE)
(72) Inventor: VANDERBIST, Francis, B-1650 Beersel (BE); SERENO, Antonio, B-1820 Melsbroek (BE); BAUDIER, Philippe, B-1180 Uccle (BE)
(74) Representative: Powis de Tenbossche, Roland
(86) International application number: PCT/BE2002/000095
(87) International publication number: WO 2002/100394

(56) References cited:
- WO-A-00/24380
- WO-A-00/37057
- WO-A-00/57859
- WO-A-00/57918
- WO-A-00/76482
- WO-A-95/24893
- WO-A-99/36060

## Description

### ABSTRACT

Oral stable liquid pharmaceutical compositions comprising at least a statin suspended in at least one water free oily excipient. The said composition allows to obtain a high bioavailability of the drug after oral administation to mammals, while reducing the formation of degradation products of the statin. A process for manufacturing the said compositions

### INTRODUCTION

Hypercholesterolaemia plays a crucial role in the development of arteriosclerosis diseases in general and coronary heart disease in particular. The risk of progression of the arteriosclerosis process to coronary heart diseases increases progressively with increasing levels of total serum cholesterol or low-density lipoproteins (LDL) cholesterol at both the individual and the population level.
The statins are reversible inhibitors of the microsomal enzyme HMG-CoA reductase, which converts HMG-CoA to mevalonate. This is an early rate-limiting step in cholesterol biosynthesis. In the present document the term"statin" and the term "HMG-CoA reductase inhibitor" are synonyms and interchangeable since both terms describe the same active ingredients.
Inhibition of HMG-CoA reductase by statins decreases intracellular cholesterol biosynthesis, which then leads to transcriptionally upregulated production of microsomal HMG-CoA reductase at cell surface LDL receptors. Subsequently, additional cholesterol is provided to the cell by de novo synthesis and by receptor-mediated uptake of LDL-cholesterol from the blood. This resets intracellular cholesterol homeostasis in extrahepatic tissues, but has little effect on the overall cholesterol balance (Clin. Pharmacokinet. 1997, May, 32(5), 403-425).
The main statins currently used in therapeutics are: pravastatin, simvastatin, lovastatin, fluvastatin, atorvastatin and cerivastatin. Lovastatin, simvastatin and pravastatin are derived from fungi (14,15).

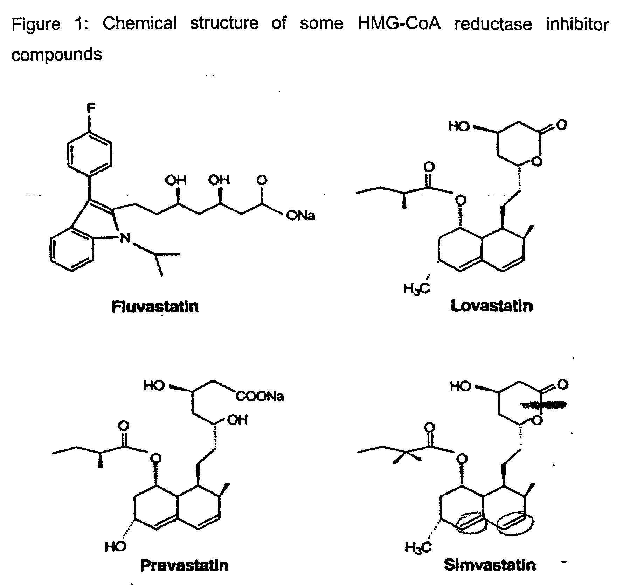

Simvastatin is a clinically modified 2,2-dimethyl-butyrate analogue of lovastatin. Pravastatin is a purified active metabolite of mevastatin with an open hydroxyacid instead of a lactose ring.
Fluvastatin and atorvastatin have an entirely synthetic origin with a structure distinct from that of the other agents (117).
The attached figure 1 shows the chemical structure of some statin molecules

The different statin molecules have widely different physico-chemical properties (Table 1). For instance, their solubility in water is widely different as shown by their partition coefficients (logarithm of partition ratio between octanol and water at pH 7.0), which range from -0.23 to 4.7 (21).

**Table 1. Physiochemical properties of the HMG-CoA reductase inhibitors fluvastatin, lovastatin, pravastatin and simvastatin**

| Property | Fluvastatin | Lovastatin | | Pravastatin | | Simvastatin | |
|---|---|---|---|---|---|---|---|
| | | Lactone | Hydroxy acid | Lactone | Hydroxy acid | Lactone | Hydroxy acid |
| M (g/mol) | 416 | 405 | 405 | 447 | 447 | 419 | 419 |
| pKa | 5.5 (acid) | | 5.5 | | 5.5 | | 5.5 |
| D | 1.5 | 4.3 | 1.7 | -0.23 | | 4.7 | 2.1 |
| Solubility in water | 2 | 0.0013 | | 0.18 | 300 | 0,0014 | |
| (g/L)^{a} | | | | | | | |
| Vₛₒₗ(ml) | 10 | 15385 | | 111 | 0.07 | 14 286 | |

| | | | | | | | |
|---|---|---|---|---|---|---|---|
| a At pH 5, except for fluvastatin at pH 6. *Abbreviations and symbols :* D=partition coefficient (logarithm of partition ratio between octanol and water at pH 7); M=molecular weight; Vₛₒₗ= volume of water require to completely dissolve a 20 mg dose at the solubility given in the table. | | | | | | | |

Those values mean that lovastatin and simvastatin are lipophilic and dissolve about 40,000 to 50,000 times more readily in octanol than in water. Pravastatin dissolves twice as readily in water, whereas fluvastatin and the hydroxyacid forms of lovastatin and simvastatin dissolve 32, 50 and 126 times more readily in octanol, respectively.
All the active compounds are acid, and have pKa values of approximately 5.5.
As summarized in table 2, the pharmacokinetic profiles of the statins are also widely different, but the different statins present the common properties of having a large intra and interindividual variability when they are taken by the oral route.

**Table 2. Intestinal absorption and systemic availability in healthy humans (unless otherwise stated) of HMG-CoA reductase inhibitors fluvastatin, lovastatin, pravastatin and simvastatin**

| Property | Fluvastatin | Lovastatin | Pravastatin | Simvastatin |
|---|---|---|---|---|
| | | (hydroxyacid) | | (hydroxy acid) |
| Oral dose (mg) | 20 | 20 | 20 | 40 |
| AUC (µg•h/L) | 167 | 15.9 | 90.2 | 58.1 |
| Cₘₐₓ (µg/L) | 181 | 3.1 | 38.4 | 58.1 |
| tₘₐₓ (h) | 0.5-1.5 | 2.8 | 1.1 | 1.2 |
| fₐ (%) | >90 | ~31 | ~34 | 60-80^{a} |
| P_{eff} (x10⁻⁴ cm/sec) | 2.4 | | | |
| F(%) | 10-35 | | 18 | |
| E_{H} (%) | 67 | 62^{a} | 45 | 80^{a} |

| | | | | |
|---|---|---|---|---|
| a From dogs *Abbreviations and symbols:* AUC = area under plasma concentration-time curve; Cₘₐₓ = maximum plasma drug concentration; E_{H} = liver extraction ratio; F = bioavailability; fₐ = fraction of the administered dose absorbed; P_{eff} = effective membrane permeability; tₘₐₓ = time to Cₘₐₓ. | | | | |

As said hereinabove, HMG CoA reductase inhibitors have very different physicochemical characteristics.
Chemically, HMG CoA reductase inhibitors can be ranged in two categories: the first category involves the molecules containing a lactose function (lovastatin, simvastatin). The molecules are inactive and need to be metabolized by esterase in the hydroxy-acid form to be active (prodrug). The second category contains molecules available under the form of the hydroxyacid (or salt). This category involves among others pravastatin sodium and fluvastatin sodium.
All the HMG CoA reductase inhibitors are relatively unstable but the molecules containing a lactose function appear particularly unstable.
Indeed, the lactose function is very easily hydrolysed and this reaction is still catalyzed by several parameters like oxygen, humidity, acidity, alkalinity and temperature. Therefore it would be of particular interest to dispose of a stable form of HMG CoA reductase inhibitors, easy to manufacture and presenting a good bioavailability of the drug
Consequently the main challenges for formulating a pharmaceutical composition comprising a statin derivative is to obtain a stable pharmaceutical composition with a high bioavailability after oral administration to humans.

### STATE OF THE ART

Little information is published about the efforts made in terms of pharmaceutical formulation on statin molecules.
A number of patents have nevertheless been granted.

The US Patent 5,180,589 describes a pharmaceutical composition which has excellent stability, when dispersed in water has a pH of at least about 9, and includes a drug which is sensitive to a low pH environment such as pravastatin, one or more fillers such as lactose and/or microcrystalline cellulose, one or more binders, such as microcrystalline cellulose or polyvinylpyrrolidone, one or more disintegrating agent such as croscarmellose sodium, one or more lubricant such as magnesium stearate and one or more basifying agent such as magnesium oxide.

The US Patent 5,356,896 describes a pharmaceutical dosage form comprising an HMG-CoA reductase inhibitor compound, e.g. fluvastatin sodium, which is stabilized against pH-related degradation by an alkaline stabilizing medium capable of maintaining at pH of at least 8 to an aqueous solution or dispersion of the composition.

The US Patent 6,235,311 describes a pharmaceutical composition which is useful for cholesterol lowering and reducing the risk of myocardial infraction, which includes a statin, such as pravastatin, lovastatin, simvastatin, or atorvastatin, cerivastatin or fluvastatin, in combination with aspirin, in a manner to minimize interaction of aspirin with the statin and minimize side effects of aspirin. A method for lowering cholesterol and reducing risk of a myocardial infraction employing such composition is also provided.

The US Patent 5,225,202 describes an enteric coated pharmaceutical composition which includes a medicament which is sensitive to a low pH environment of less than 3, such as pravastatin, which composition is preferably in the form of pellets which include an enteric coating formed of neutralized hydroxypropylmethylcellulose phthalate, plasticizer and anti-adherent. The so coated pellets have good resistance to deterioration at pH less than 3 but have good drug release properties at greater than 3.

The WO 00/76482 patent describes a pharmaceutical form with increased bioavailability obtained by solubilizing a lipid-regulating agent in one or more components, then solidifying it by adding one or more solid or semi-solid constituents.
The main problem met with this kind of formulations is that heating the formulation to at least 50°C is required, what can provoke a significant degradation of the statin derivatives.

Furthermore, the amount of excipients needed for dissolving the active ingredient is very important, what makes the final pharmaceutical form very big and consequently very difficult to administer to humans.

The WO 00/57918 patent is directed to a formulation comprising a lipid-regulating agent dissolved in a mixture of an oil and one or more surfactants. The concentrate forms a fine emulsion when water is added in the composition.
The main disadvantage of the invention hereinabove is that the presence of water in a composition is often incompatible with the capsule in which the formulation must by filled to form the final pharmaceutical form. On the other hand, the amount (or volume of excipients) needed to provide the adequate formulation is very important and makes the pharmaceutical form particularly difficult to administer to patients.

The WO 00/57859 patent is directed to a formulation comprising a lipid-regulating agent in at least one oil and one emulsifier blend, then the resulting mixture is transformed in an emulsion upon dilution in an aqueous medium.
The main disadvantage of this invention is again the compatibility of water with the capsule shell and/or the other excipients and/or active ingredients. For instance, the statin derivatives possessing a lactose group will be very rapidly hydrolyzed in the presence of water.
Furthermore, the quality of excipients needed to obtain the final pharmaceutical form does not allow the manufacturing of a practical, easy-to-swallow form for the patient.
Indeed, enormous amounts (or volumes) of excipients are needed to make the final pharmaceutical form.

### SUMMARY OF THE INVENTION

It is an object of the present invention to provide a stable pharmaceutical composition containing a statin derivative. It is also an object of the present invention to provide a composition presenting a high bioavailability of the drug and/or its active metabolite(s) after oral administration to humans. It is another object of the present invention to provide a safe, cheap, ecological rapid and easy process to manufacture the said composition.

### BRIEF DESCRIPTION OF THE DRAWINGS

Figure 1 gives the Chemical structure of some HMG-CoA reductase inhibitor compounds.

### BACKGROUND OF THE INVENTION

it was discovered that liquid oily formulations could be an interesting alternative for the formulation of HMG CoA reductase inhibitors both in term of stability of the drug in the final composition and in term of the bioavailability of the drug in humans.
It is also a purpose of the present invention to describe a process for manufacturing a pharmaceutical composition, which process is ecological, economically advantageous, rapid, simple and safe for the operators.

The present invention relates to a liquid oily pharmaceutical composition containing at least one HMG-CoA reductase inhibitor agent.

The liquid suspension contains advantageously at least one oily excipient and one stabilizing agent e.g. one or more antioxidant or preservative agent(s) or combination of both preservative and antioxidant agents.

The oral pharmaceutical formulation of the invention comprises at least one pharmaceutically acceptable carrier and a water free suspension of solid particles containing at least one statin with a particle size of less than 500µm (advantageously lower than 200µm) in at least one pharmaceutical oil or mix of pharmaceutically acceptable oils. The oil or mix of oils is liquid at room temperature (20°C), preferably at temperature below 20°C, such as temperature below 10°C, or even at temperature below 0°C. The viscosity of the suspension is advantageously controlled so that said viscosity is lower than 100 mPa·s at 20°C, advantageously lower than 50 mPa·s at 20°C.
The pharmaceutically acceptable carrier is adapted for releasing said water free suspension in the gastric tract and/or in the intestine tract, especially in the intestine tract and/or controlling the release of said water free suspension in the gastric tract and/or in the intestine tract, especially in the intestine tract. It means that when a patient is orally administered with a formulation of the invention, the water free oily suspension of solid statin particles is liberated as a suspension in the gastric tract and/or intestine tract. The carrier is for example a capsule containing the suspension, said capsule being dissolved and/or disintegrated and/or destroyed in the gastric and/or intestine tract, whereby ensuring the release of the water free oily suspension in the gastric and/or intestine tract.

The oral formulation of the invention comprises advantageously an amount of statin corresponding to at least 1,5%, advantageously at least 3%, preferably at least 4% by weight of the total weight of the water free oily suspension, preferably of the oral formulation.

According to preferred embodiment, the solid particles containing at least one statin have a weight average particles size lower than 100µm, advantageously lower than 50µm, preferably lower than 50µm, most preferably comprised between 2 and 30µm, such as about 10µm or about 20µm.
According to a detail of an embodiment, the water free suspension comprises about 0.5 to 50 % of at least one statin and about 30 % to 99% of a pharmaceutical oil or mix of oils liquid at room temperature.
The statin is for example selected from the group consisting of simvastatin, lovastatin, fluvastatin, atorvastatin, cerivastatin, pravastatin and combinations of several of those molecules.
The pharmaceutical oil or mix of oils is advantageously selected from the group consisting of soyabean oil, olive oil, peanut oil, arachide oil, paraffine, a mono-, di, or triglyceride of C6-C18 fatty acids, polyethyleglycol derivatives, and mixtures of several of those oils
The suspension can further comprise at least one pharmaceutically acceptable excipient selected from the group consisting of antioxidants, stabilizers, viscosifiers, emulsifiers, surfactants and mix of several of those excipients.
The suspension advantageously further comprises at least_ one pharmaceutically acceptable antioxidant or a combination of antioxidants selected from the group consisting of Vitamine E derivatives, methoxyphenol derivatives such as butylhydroxyanisole, butylhydroxytoluene, propyl gallate and mixtures thereof.
As the oral formulation of the invention is extremely stable, the suspension comprises substantially no degradation product or products of the statin after 1 month storage at 25°C in a relative air humidity of 60%, the weight ratio degradation product or products/statin being advantageously lower than 0.5, preferably lower than 0.2 after 1 month storage at 25°C in a relative air humidity of 60%. For example, the weight ratio degradation product or products/statin is advantageously lower than 0.5, preferably lower than 0.2 after at least 2 months (such after 3 months or even after 6 months) storage at 25°C in a relative air humidity of 60%.
According to a detail of an embodiment, the suspension is filled into pharmaceutically acceptable capsules.
The oral formulation comprises preferably a pharmaceutical dose of statin suitable for administration to humans.
The suspension comprises preferably an oily phase composed by a mix of soyabean oil and medium chain triglycerides, said mix being advantageously free of emulsifiers and surfactants.
The suspension preferably comprises one or more vitamine E derivative(s) combined with one or more methoxyphenol derivative(s) or with other acceptable antioxydants.
The invention relates also to a manufacturing process for the preparation of an oral formulation of the invention, in which a suspension of statin in a water free liquid containing one or more pharmaceutically acceptable oils is prepared, and in which the so prepared water free suspension is filled in a carrier.
The process of claim 15, in which a water free and statin free oil liquid is prepared by mixing at least one or more pharmaceutically acceptable oils, with one or more antioxydants (advantageously without emulsifier or surfactant), and in which particles of statin are mixed with the water free liquid up to the preparation of a homogeneous water free suspension.

The way to discover that water free oily liquid formulations of the invention was the best pharmaceutical form for statin derivatives both in terms of stability and bioavailability has been long and surprising.

Indeed, in a first step, we have formulated a statin derivative (simvastatine) as a tablet form. Two tablets have been formulated, one being a direct compression tablet, (batch: 7B2001/B), the other a tablet made after a wet granulation process (batch: 06D2001).

The tablets manufactured by direct compression (batch 7B2001 B) contained as inactive ingredient : lactose monohydrate, povidone, crospovidone, citric acid and magnesium stearate and the tablets made by wet granulation (batch 06D2001) contained as inactive ingredient : lactose monohydrate, microcrytalline cellullose, povidone, citric acid and magnesium stearate. Both formulations are classic and contain well known and widely described inactive ingredients.

Both tablet formulations have been administered to 6 human volunteers in a single dose, cross-over pharmacokinetic study. The reference formulation was ZOCOR^{®} 40 mg (Merck, Sharp & Dohme). The formulation of the tablets is given hereinbelow: It should be noted that, for a better understanding of the present invention, all the pharmacokinetic data given are normalized to 40 mg for simvastatin. Furthermore, all the pharmacokinetic data are expressed as a percentage obtained by dividing the value obtained with reference drug and the percentage obtained with new drug developed. Consequently, a ratio of 100 % described bioequivalent products, a ratio > 100 % described a bioavailability superior for the reference drug while a ratio < 100 % described a bioavailability superior for the new drug making the object of the invention
The results of the pharmacokinetic study clearly indicate that the bioavailability of simvastatin after the two formulated tablets was lower than with the reference.

**Table 3 : comparative pharmacokinetic data of simvastatin and its active metabolite 4-hydroxy simvastatin after oral administration to 6 healthy volunteers of two tablets formulations versus Zocor^{®}**

| | Zocor | 7B2001B | 06D2001 |
|---|---|---|---|
| SIMVASTATIN (ratio reference / test) | | | |
| AUC (0-12h) (ng.h/ml) | 100 | 110 | 209 |
| Cmax (ng/ml) | 100 | 162 | 301 |

| 4-HYDROXY-SIMVASTATIN (ratio reference / test) | | | |
|---|---|---|---|
| AUC (0-12h) (ng.h/ml) | 100 | 123 | 163 |
| Cmax (ng/ml) | 100 | 148 | 179 |

It can be observed that both tablets tested present ratio of AUC and Cmax very significantly higher than 100 %. That means that the tablets developed present a lower bioavailability of simvastatin and its active metabolite 4-hydroxy simvastatin than the reference
it can be concluded that the formulation and/or manufacturing process of simvastatin has a great influence on its bioavailability after oral administration to humans. It can also be concluded that it is not obvious for a man skilled in the art to obtain a pharmaceutical form presenting a high bioavailability of simvastatin and / or its active metabolite 4-hydroxy simvastatin, since classical formulations give very low results.

Ina second step,it was decided to formulate the statin derivative as a semi-solid form in which the statin would be at least partially dissolved to increase the bioavailability of the drug. Different compositions have been formulated using for instance polyglycolized glycerides (Gelucire^{®}) as solubilizing agents. Different examples of such compositions are given hereinbelow.

**Table 4 : examples of semi-solid formulation of simvastatin**

| Ingredients | 25F2001B | 25F2001A | 22H2001 |
|---|---|---|---|
| simvastatin | 40 | 40 | 40 |
| Gelucire 44/14 | 350 | | / |
| Gelucire 50/13 | / | 583 | 583 |
| Myverol | / | / | / |
| Transcutol | / | / | / |
| Vit E TPGS | 30 | 50 | 50 |
| PEG 600 | / | / | 33 |
| butylhydroxyanisole | 0.04 | 0.04 | 0.04 |
| butylhydroxytoluene | 0.04 | 0.04 | 0.04 |

A pharmacokinetic study was performed on 6 volunteers with the formulations given hereinabove and compared to ZOCOR^{®} 40 mg (Merck, Sharp and Dohme) as reference product, and as it can be observed hereinbelow, the pharmacokinetic parameters had significantly increased for the semi-solid formulation in comparison with the reference product since the ratio {(reference / new formulation) X 100} is significantly inferior to 100 %.

**Table 5 : comparative pharmacokinetic data of simvastatin and its active metabolite 4-hydroxy simvastatin after oral administration to 6 healthy volunteers**

| | Zocor | 25F2001B | 22H2001 |
|---|---|---|---|
| SIMVASTATIN (ratio reference / test) | | | |
| AUC (0-12h) (ng.h/ml) | 100 | 87 | 63 |
| Cmax (ng/ml) | 100 | 70 | 39 |

| 4-HYDROXY-SIMVASTATIN (ratio reference / test) | | | |
|---|---|---|---|
| AUC (0-12h) (ng.h/ml) | 100 | 83 | 50 |
| Cmax (ng/ml) | 100 | 90 | 54 |

Unfortunately, it was found out that the stability data on the semi-solid formulations of simvastatin show a significant instability at 25°C/60% relative humidity after 1 month storage. This instability is principally characterized by the apparition of the main degradation product of simvastatin, the 4-hydroxy simvastatin The stability data are given hereinbelow

**Table 6 : stability of semi-solid formulations of simvastatin**

| | % 4-OH simvastatin | | |
|---|---|---|---|
| | 25F2001B | 25F2001A | 22H2001 |
| | (55°C)* | (38 °C) | (40°C) |
| Initial | 0.14 | 0.17 | 0.15 |
| 1 month 25°C / 60 % RH | 4.31 | 1.5 | 1.43 |

| | | | |
|---|---|---|---|
| * temperature at which the composition is heated to allow the capsule filling | | | |

This instability may be provoked by the heating of the formulation necessary to make the composition liquid and hence allow the capsule filling.
We decreased the temperature to 38°C (25F2001A) during the manufacturing process but the instability of simvastatin after 1 month storage at 25°C/60% RH was still significant.

We then decided and this is the object of the present invention to formulate the statin derivative as a liquid suspension in an oil to assess its stability. Different compositions of simvastatin as such suspension were formulated, containing at least the statin derivative, one oily vehicle and one or more antioxydant and/or stabilizing agents. The formulations were manufactured without heating or in other words at the ambient temperature.

**Table 7 : examples of formulations of statins in oily suspensions**

| Ingredients | 1B2002 | 6L2001 | 17A2002 |
|---|---|---|---|
| simvastatin | 40 | 40 | 40 |
| soya bean oil | 527 | 310 | 585 |
| medium chain triglycerides | 317 | / | 325 |
| Vit E acetate | / | / | 17 |
| Vit E TPGS | / | / | 33 |
| PEG 600 | / | 483 | / |
| butylhydroxyanisole | 0.07 | 0.07 | 0.07 |
| butylhydroxytoluene | / | 0.07 | / |
| propylgallate | 0.07 | / | 0.07 |

The stability of those suspensions after 1 month storage at 25°C/60% RH did not show any significant apparition of degradation products.

**Table 8 : stability of semi-solid formulations of simvastatin**

| | % 4-OH simvastatin | | |
|---|---|---|---|
| | 17402 | 8D2002 | 06L2001 |
| Initial | 0.011 1 | 0.022 | 0.015 |
| 1 month 25°C / 60 % RH | 0.074 | 0.044 | 0.085 |

We then decided to perform a cross-over pharmacokinetic study with those suspensions on 6 healthy volunteers. The reference drug was ZOCOR^{®} 40 mg. Suprisingly enough, while simvastatin was formulated as a suspension (and not an emulsion or a micellar solution), the bioavailability of the drug had increased in comparison with the reference. Indeed, the pharmacokinetic parameters show a high suprabioavailability of the composition described in the present invention when compared to the reference product. This is surprising since simvastatin having a very poor water solubility, it was not expected that a suspension could offer such a high bioavailability. Indeed, for this kind of drugs, the solubility is often the limiting step for the resoprtion of the drug.

**Table 9 : comparative pharmacokinetic data of simvastatin and its active metabolite 4-hydroxy simvastatin after oral administration to 6 healthy volunteers**

| | Zocor | 17A02 | 6L2002 |
|---|---|---|---|
| SIMVASTATIN (ratio reference / test) | | | |
| AUC (0-12h) (ng.h/ml) | 100 | 86 | 96 |
| Cmax (ng/ml) | 100 | 98 | 95 |

| 4-HYDROXY-SIMVASTATIN (ratio reference test) | | | |
|---|---|---|---|
| AUC (0-12h) (ng.h/ml) | 100 | 57 | 73 |
| Cmax (ng/ml) | 100 | 56 | 65 |

It can be observed from thet data given hereinabove that the suprabioavailability of the composition making the object of the present invention is more marked on the active metabolite (4-OH-simvastatin) than on the prodrug simvastatin.

The oily liquid composition is typically a suspension. This has for instance be proven by comparing the measurement of the particle size of simvastatin as raw material (measured by laser diffraction) and the particle size of simvastatin in the oily suspension (measured by hot stage microscopy). Both measures give a mean particle size of about 20 µm. This means that the simvastatin is neither dissolved nor emuslified in the oily excipients. Indeed, since the particle size of the raw material is maintained in the final composition, the composition is a suspension. Furthermore, the crystals of simvastatin in the oily suspensions can be observed very easily with an optical microscope.

The oily vehicle suitable to suspend the statin derivative may be but is not limited to soyabean oil, olive oil, peanut oil, arachide oil, paraffine, a mono, di, or triglyceride of a C6-C18 fatty acid, a polyethyleneglycol derivative or a mix of several of those oils.
Also advantageous for the stability and, in some cases, the bioavailability of the composition is the addition of an antioxidant agent such as, but not limited to, a Tocopherol derivative like α Tocopherol (Vitamin E), α Tocopherol acetate, Vitamin E TPGS or a methylphenol derivative like butylhydroxyanisol (BHA) or butylhydroxytoluene (BHT).
Although some examples of formulations have been given in table 7, some detailed examples are given hereinbelow to clearly illustrate the invention.

### Examples

1) 585 g of soya bean oil are mixed together with 325 g of medium chain triglycerides in an adequate mixer (for instance a Bi-Agi^{®} 2.5 liters Lleal, Spain) and at ambient temperature. 33 g of vitamine liquid E TPGs are added slowly and under agitation to the oily vehicle. Then 17 g of acetate of tocopherol are added. 0.07 g of butylhydroxyanisole and 0.07 g of propylgallate are added to the mix. The mixing is maintained until complete dissolution of the antioxidants. Finally, 40 g of simvastatin are added to the mix and the mixing is maintained until completed homogeneisation of the simvastatin suspended in the oily vehicle. The oily mix is then filled into hard gelatin capsules and the capsules are sealed.
2) 310 g of soya bean oil are mixed together with 483 g of polyethyleneglycol 600 in an adequate mixer (for instance a Bi-Agi^{®} 2.5 liters Lleal, Spain) and at ambient temperature. 0.07 g of butylhydroxyanisole and 0.07 g of propylgallate are added to the mix. The mixing is maintained until complete dissolution of the antioxydants. Finally, 40 g of simvastatin are added to the mix and the mixing is maintained until completed homogeneisation of the simvastatin suspended in the oily vehicle. The oily mix is then filled into hard gelatin capsules and the capsules are sealed.
3) 400 g of medium chain triglycerides in filled in an adequate mixer (for instance a Bi-Agi^{®} 2.5 liters Lleal, Spain) at ambient temperature. 70 g of vitamine liquid E TPGs are added slowly and under agitation to the oily vehicle. 0.07 g of butylhydroxyanisole and 0.07 g of propylgallate are added to the mix. The mixing is maintained until complete dissolution of the antioxydants. Finally, 40 g of pravastatin Na are added to the mix and the mixing is maintained until completed homogeneisation of the simvastatin suspended in the oily vehicle. The oily mix is then filled into hard gelatin capsules.
4) Detailed example of manufacturing process of the said pharmaceutical composition.

One of the advantages of the invention relates to the easiness of the manufacturing process of the medication and the rapidity and easiness of the pharmaceutical composition.
- Mix soya bean oil with Akomed^{®} at ambient temperature in an adequate mixing tank (Lleal tri-agi^{®} or fryma^{®})
- Add the liquid Vit E TPGS^{®}; maintain the mix below 30°C
- Add the antioxidants butylhydroxianisole and butylhydroxitoluene
- Maintain the mixing until complete solubilisation of the antioxidant
- Add simvastatin and allow homogenization of the drug
- Fill into hard gelatin or hypromellose capsule
- Seal the capsule (optional)
- Package into blister of HDPET bottle

This manufacturing process presents the advantages to be quite short, ecological, simple, safe for the operators and is very easily applicable at an industrial scale. Furthermoren, no heating is required what is very advantangeous for the stability of statins derivatives.

## Claims

1. An oral pharmaceutical formulation comprising at least one pharmaceutically acceptable carrier and a water free suspension of solid particles containing at least one statin with a particle size of less than 500µm in at least one pharmaceutical oil or mix of oils, whereby said oil or mix of oils is liquid at room temperature (20°C), in which said pharmaceutically acceptable carrier is adapted for releasing said water free suspension in the gastric tract and/or in the intestine tract.

2. The oral formulation of claim 1, which comprises a amount of statin corresponding to at least 1,5%, advantageously at least 3%, preferably at least 4% by weight of the total weight of the water free suspension, preferably of the formulation.

3. The oral formulation of claim 1, in which the solid particles containing at least one statin have a particle size of less than 200µm.

4. The oral formulation of claim 1, in which the solid particles containing at least one statin have a weight average particles size lower than 100µm, advantageously lower than 50µm, preferably lower than 50µm, most preferably comprised between 2 and 30µm.

5. The oral formulation of claim 1, in which the water free suspension comprises 0.5 to 50 % of at least one statin and 30 % to 99% of a pharmaceutical oil or mix of oils liquid at room temperature.

6. The oral formulation of claim 1, in the statin is selected from the group consisting of simvastatin, lovastatin, fluvastatin, atorvastatin, cerivastatin, pravastatin and combinations of several of those molecules.

7. The oral formulation of claim 1, in which the pharmaceutical oil or mix of oils is selected from the group consisting of soyabean oil, olive oil, peanut oil, arachide oil, paraffine, a mono-, di, or triglyceride of C6-C18 fatty acids, polyethyleneglycol derivatives, and mixtures of several of those oils

8. The oral formulation of claim 1, in which the suspension further comprises at least one pharmaceutically acceptable excipient selected from the group consisting of antioxidants, stabilizers, viscosifiers, emulsifiers, surfactants and mix of several of those excipients.

9. The oral formulation of claim 1, in which the suspension further comprises at least one pharmaceutically acceptable antioxidant or a combination of antioxidants selected from the group consisting of Vitamine E derivatives, methoxyphenol derivatives such as butylhydroxyanisole, butylhydroxytoluene, propyl gallate and mixtures thereof.

10. The oral formulation of claim 1, in which the suspension comprises substantially no degradation product or products of the statin after 1month storage at 25°C in a relative air humidity of 60%, the weight ratio degradation product or products/statin or statin derivative being advantageously lower than 0.5, preferably lower than 0.2 after 1 month storage at 25°C in a relative air humidity of 60%.

11. The oral formulation of claim 1, in which the suspension is filled into pharmaceutically acceptable capsules.

12. The oral formulation of claim 1, which comprises a pharmaceutical dose of statin suitable for administration to humans.

13. The oral formulation of claim 1, in which the suspension comprises an oily phase composed by a mix of soyabean oil and medium chain triglycerides.

14. The oral formulation of claim 1, which the suspension comprises one or more vitamine E derivative(s) combined with one or more methoxyphenol derivative(s) or with other acceptable antioxydants.

15. The oral formulation of claim 1, in which the water free suspension is free of emulsifier or surfactant.

16. A manufacturing process for the preparation of an oral formulation of anyone of the preceding claims, in which a suspension of statin in a water free liquid containing one or more pharmaceutically acceptable oils is prepared, and in which the so prepared water free suspension is filled in a carrier.

17. The process of claim 15, in which a water free and statin free oil liquid is prepared by mixing one or more pharmaceutically acceptable oils, one or more antioxydants and one or more emulsifiers or surfactant, and in which particles of statin are mixed with the water free liquid up to the preparation of a homogeneous water free suspension.

## Patentansprüche

1. Orales pharmazeutisches Präparat umfassend mindestens einen pharmazeutisch verträglichen Träger und eine wasserfreie Suspension von festen Partikeln mit mindestens einem Statin mit einer Partikelgröße von weniger als 500 µm in mindestens einem pharmazeutischen Öl oder Ölgemisch, wobei dieses Öl oder Ölgemisch bei Raumtemperatur (20°C) flüssig ist, in dem der pharmazeutisch verträgliche Träger so angepaßt ist, dass er die wasserfreie Suspension von festen Partikeln im Magen- und/oder Darmtrakt freisetzt.

2. Das orale Präparat nach Anspruch 1, das eine Statinmenge enthält, die mindestens 1,5 , vorteilhafterweise mindestens 3, vorzugsweise mindestens 4 Gewichtsprozent des Gesamtgewichts der wasserfreien Suspension, bevorzugt des Präparats entspricht.

3. Das orale Präparat nach Anspruch 1, in dem die mindestens ein Statin enthaltenden Partikel eine Partikelgröße von weniger als 200 µm haben.

4. Das orale Präparat nach Anspruch 1, in dem die mindestens ein Statin enthaltenden Partikel eine mittlere Partikelgröße von weniger als 100 µm, vorteilhafterweise von weniger als 50 µm, bevorzugt von weniger als 50 µm, am meisten bevorzugt zwischen 2 und 30 µm haben.

5. Das orale Präparat nach Anspruch 1, in dem die wasserfreie Suspension 0,5 bis 50 % von mindestens einem Statin und 30 bis 99 % eines bei Raumtemperatur flüssigen pharmazeutischen Öls oder Ölgemischs umfasst.

6. Das orale Präparat nach Anspruch 1, in dem das Statin ausgewählt ist aus der Gruppe bestehend aus Simvastatin, Lovastatin, Fluvastatin, Atorvastatin, Cerivastatin, Pravastatin und Kombinationen von mehreren dieser Moleküle.

7. Das orale Präparat nach Anspruch 1, in dem das pharmazeutische Öl oder Ölgemisch ausgewählt ist aus der Gruppe bestehend aus Sojabohnenöl, Olivenöl, Erdnussöl, Arachisöl, Paraffin, einem Mono-, Di- oder Triglycerid von C6 - C18 Fettsäuren, Polyethylenglykolderivaten und Gemischen von mehreren dieser Öle.

8. Das orale Präparat nach Anspruch 1, in dem die Suspension weiterhin mindestens ein pharmazeutisch verträgliches excipient ausgewählt aus der Gruppe bestehend aus Antioxidatien, Stabilisatoren, Verdickern, Emulgatoren, oberflächenaktiven Stoffen und Mischungen von mehreren dieser Bindemittel umfasst.

9. Das orale Präparat nach Anspruch 1, in dem die Suspension weiterhin mindestens ein pharmazeutisch verträgliches Antioxidationsmittel oder eine Kombination von Antioxidationsmitteln enthält, die ausgewählt sind aus der Gruppe bestehend aus Vitamin-E-Derivaten, Methoxyphenolderivaten wie Butylhydroxyanisol, Butylhydroxytoluen, Propylgallat und Gemischen daraus.

10. Das orale Präparat nach Anspruch 1, in dem die Suspension nach 1 Monat Lagerung bei 25 °C und einer relativen Luftfeuchtigkeit von 60 % im Wesentlichen kein Abbauprodukt oder Abbauprodukte des Statins umfasst und bei dem das Masseverhältnis Abbauprodukt oder Abbauprodukte/Statin oder Statinderivat nach 1 Monat Lagerung bei 25 °C und einer relativen Luftfeuchtigkeit von 60 % bevorzugt weniger als 0,5, vorzugsweise weniger als 0,2 beträgt.

11. Das orale Präparat nach Anspruch 1, in dem die Suspension in pharmazeutisch verträgliche Kapseln gefüllt ist.

12. Das orale Präparat nach Anspruch 1, das eine pharmazeutische Dosis eines Statins umfasst, die für die Verabreichung an Menschen geeignet ist.

13. Das orale Präparat nach Anspruch 1, in dem die Suspension eine Ölphase umfasst, die aus einem Gemisch aus Sojabohnenöl und mittelkettigen Triglyceriden besteht.

14. Das orale Präparat nach Anspruch 1, in dem die Suspension mindestens ein oder mehrere Vitamin-E-Derivate in Kombination mit mindestens einem oder mehreren Methoxyphenolderivaten oder anderen verträglichen Antioxidantien umfasst.

15. Das orale Präparat nach Anspruch 1, in dem die wasserfreie Suspension frei ist von Emulgatoren oder oberflächenaktiven Stoffen.

16. Herstellungsverfahren zur Herstellung eines oralen Präparats nach einem der vorhergehenden Ansprüche, bei dem eine Statinsuspension in einer wasserfreien Flüssigkeit mit einem oder mehreren pharmazeutisch verträglichen Ölen hergestellt wird, und bei dem die so hergestellte wasserfreie Suspension in einen Träger gefüllt wird.

17. Das Verfahren nach Anspruch 16, bei dem durch Mischen von einem oder mehreren pharmazeutisch verträglichen Ölen, von einem oder mehreren Antioxidantien und von einem oder mehreren Emulgatoren oder oberflächenaktiven Stoffen eine wasser- und statinfreie Ölflüssigkeit hergestellt wird, und bei dem Statinpartikel mit der wasserfreien Flüssigkeit gemischt werden bis zur Herstellung einer homogenen wasserfreien Suspension.

## Revendications

1. Formulation pharmaceutique orale comprenant au moins un support pharmaceutiquement acceptable et une suspension sans eau de particules solides contenant au moins une statine avec une taille de particule de moins de 500 µm dans au moins une huile pharmaceutique ou un mélange d'huiles, par quoi ladite huile ou mélange d'huiles est liquide à température ambiante (20°C), dans laquelle ledit support pharmaceutiquement acceptable est adapté pour libérer ladite suspension sans eau dans le tractus gastrique et/ou le tractus intestinal.

2. Formulation orale selon la revendication 1, qui comprend une quantité de statine correspondant au moins à 1,5%, avantageusement au moins à 3%, de préférence au moins à 4% en poids du poids total de la suspension sans eau, de préférence de la formulation.

3. Formulation orale selon la revendication 1, dans laquelle les particules solides contenant au moins une statine ont une taille de particule de moins de 200 µm.

4. Formulation orale selon la revendication 1, dans laquelle les particules solides contenant au moins une statine ont une taille de particule moyenne en poids inférieure à 100 µm, avantageusement inférieure à 50 µm, de préférence inférieure à 50 µm, davantage préféré comprise entre 2 et 30 µm.

5. Formulation orale selon la revendication 1, dans laquelle la suspension sans eau comprend 0,5 à 50% d'au moins une statine et 30% à 99% d'une huile pharmaceutique ou d'un mélange d'huiles, liquide à température ambiante.

6. Formulation orale selon la revendication 1, dans laquelle la statine est choisie parmi le groupe constitué de la simvastatine, la lovastatine, la fluvastatine, l'atorvastatine, la cérivastatine, la pravastatine et des combinaisons de plusieurs de ces molécules.

7. Formulation orale selon la revendication 1, dans laquelle l'huile pharmaceutique ou le mélange d'huiles est choisi parmi le groupe constitué de l'huile de soja, de l'huile d'olive, de l'huile de cacahuète, de l'huile d'arachide, de la paraffine, d'une mono-, di-, ou tri-glycéride d'acide gras en C6-C18, de dérivés de polyéthylèneglycol et de mélanges de plusieurs de ces huiles

8. Formulation orale selon la revendication 1, dans laquelle la suspension comprend en plus au moins un excipient pharmaceutiquement acceptable choisi parmi le groupe constitué d'antioxydants, de stabilisants, d'additifs de viscosité, d'émulsifiants, de tensioactifs et de mélange de plusieurs de ces excipients.

9. Formulation orale selon la revendication 1, dans laquelle la suspension comprend en plus au moins un antioxydant pharmaceutiquement acceptable ou une combinaison d'antioxydants choisis parmi le groupe constitué des dérivés de Vitamine E, des dérivés de méthoxyphénol comme le butylhydroxyanisole, le butylhydroxytoluène, le gallate de propyle et des mélange de ceux-ci.

10. Formulation orale selon la revendication 1, dans laquelle la suspension ne comprend essentiellement aucun produit ou produits de dégradation de la statine après 1 mois de stockage à 25°C dans une humidité relative de l'air de 60%, le rapport en poids du produit ou des produits de dégradation/statine ou dérivé de statine étant avantageusement inférieur à 0,5, de préférence inférieur à 0,2 après 1 mois de stockage à 25°C dans une humidité relative de l'air de 60%.

11. Formulation orale selon la revendication 1, dans laquelle la suspension est remplie dans des capsules pharmaceutiquement acceptables.

12. Formulation orale selon la revendication 1, qui comprend une dose pharmaceutique de statine appropriée pour l'administration aux êtres humains.

13. Formulation orale selon la revendication 1, dans laquelle la suspension comprend une phase huileuse composée d'un mélange d'huile de soja et de triglycérides à chaîne moyenne.

14. Formulation orale selon la revendication 1, dans laquelle la suspension comprend un ou plusieurs dérivés de vitamine E combinés avec un ou plusieurs dérivés de méthoxyphénol ou avec d'autres antioxydants acceptables.

15. Formulation orale selon la revendication 1, dans laquelle la suspension sans eau est exempte d'émulsifiant ou de tensioactif.

16. Procédé de fabrication pour la préparation d'une formulation orale selon l'une quelconque des revendications précédentes, dans lequel une suspension de statine dans un liquide sans eau contenant une ou plusieurs huiles pharmaceutiquement acceptables est préparée, et dans lequel la suspension sans eau ainsi préparée est remplie dans un support.

17. Procédé selon la revendication 15, dans lequel un liquide huileux sans eau et sans statine est préparé par mélange d'une ou plusieurs huiles pharmaceutiquement acceptables, un ou plusieurs antioxydants et un ou plusieurs émulsifiants ou tensioactifs, et dans lequel les particules de statine sont mélangées avec le liquide sans eau pour la préparation d'une suspension homogène sans eau.
